# FASCICULE DE BREVET EUROPEEN

(11) **EP 4 308 022 B1**
(45) Date de publication et mention de la délivrance du brevet: **25.02.2026**
(21) Numéro de dépôt: 22726108.8
(22) Date de dépôt: 28.04.2022
(51) Int. Cl.: A61B 18/02, A61F 7/10, A61F 7/02

(54) **DISPOSITIF NOMADE ET AUTONOME DE RECUPERATION MUSCULAIRE PAR CRYOTHERAPIE**
TRAGBARE, EIGENSTÄNDIGE VORRICHTUNG ZUR MUSKELERHOLUNG MITTELS KRYOTHERAPIE
PORTABLE, STANDALONE DEVICE FOR MUSCLE RECOVERY BY MEANS OF CRYOTHERAPY

(30) Priorité: 28.04.2021 FR 2104442
(43) Date de publication de la demande: 24.01.2024
(73) Titulaire: Modelski, Guillaume, 34130 Mauguio (FR)
(72) Inventeur: Modelski, Guillaume, 34130 Mauguio (FR)
(74) Mandataire: Cabinet Netter
(86) Numéro de dépôt international: PCT/EP2022/061401
(87) Numéro de publication internationale: WO 2022/229348

(56) Documents cités:
- WO-A1-01/68185
- WO-A1-2019/023569

## Description

### Domaine technique de l'invention

La présente invention vise un dispositif nomade et autonome de récupération musculaire par cryothérapie. Elle s'applique, en particulier, à la récupération avec un effort musculaire intense, typiquement sportif et à la réduction des douleurs musculaires et des œdèmes, quelles qu'en soient les causes. Plus particulièrement, l'invention vise la récupération musculaire des différents membres du corps, notamment mollets et cuisses.

### État de la technique

Pendant que le corps produit un effort physique intense, la consommation d'oxygène par les muscles devient plus importante que l'oxygène apporté à ces muscles par les artères. En effet, l'effort musculaire réduit la pression veineuse et les contractions musculaires répétées compriment les vaisseaux nourriciers intramusculaires, ce qui limite l'apport de sang et d'oxygène.

Il s'ensuit une accumulation dans les muscles de toxines que le corps va tenter d'éliminer par une augmentation de la circulation des fluides au travers d'une réponse inflammatoire. Il en résulte une fatigue musculaire, des douleurs et des courbatures.

Dans ce contexte, le caractère bénéfique de la cryothérapie est connu, en particulier en vue de la récupération musculaire après avoir produit un effort physique important.

Cependant pour observer un effet bénéfique de la cryothérapie sur la récupération musculaire, il est nécessaire de créer un choc thermique à la surface de la peau de la zone anatomique que l'on souhaite traiter. Un tel choc thermique est obtenu lorsque la température cutanée est abaissée à une température inférieure ou égale à 0°C. Cependant, la cryothérapie impose de se déplacer dans un établissement disposant des matériels nécessaires, ce qui impose un important délai entre l'effort musculaire et l'effet cryothérapeutique et une perte de temps conséquente.

On connaît également des chaussettes qui, dans la partie autour du pied, comportent des poches à l'intérieur desquelles peuvent être placés des coussinets de gel que l'on peut, à volonté, chauffer ou refroidir, selon que l'on souhaite réchauffer ses pieds ou, au contraire, les refroidir et, ainsi, les soulager d'une douleur passagère ou chronique. Une telle chaussette, en ne s'étendant qu'autour du pied, ne peut contribuer à la récupération musculaire de la jambe d'un usager, en particulier de son mollet. De plus, cette chaussette se limite à une action sur le pied par un apport de froid et de chaud par des coussinets eux-mêmes refroidis ou réchauffés par des moyens extérieurs, qu'il est donc nécessaire de trouver. Ces chaussettes ne sont donc pas autonomes.

On connaît aussi le document US 2016/0051400, qui décrit une botte susceptible de contenir un liquide froid, notamment de l'eau glacée. Le sportif est donc en mesure d'enfiler une telle botte immédiatement en fin d'exercice physique pour entamer le processus de récupération musculaire. Cependant, cette botte présente un certain nombre d'inconvénients. L'utilisation d'eau glacée ne permet pas de créer un choc thermique suffisant pour que l'on puisse observer des bénéfices physiologiques notables. De plus, il est nécessaire de prévoir des moyens externes de refroidissement de l'eau. Si bien que ces bottes ne sont pas autonomes.

Cette botte comporte, par ailleurs, des moyens pour assurer la circulation du fluide froid autour du pied, ces moyens se présentant, en particulier, souvent sous forme d'une pompe à air. De plus, elle n'offre guère la possibilité de cibler précisément les zones de la jambe à refroidir.

On connaît la demande de brevet WO2019/023569 qui divulgue un dispositif de cryoablation, c'est-à-dire, de destruction de tissus. On connaît également la demande de brevet WO01/68185 qui divulgue un dispositif de traitement vasculaire par laser présentant une surface de refroidie pour éviter de bruler la peau d'un utilisateur au travers de laquelle, une veine est traitée.

Bien entendu, ces documents sont très éloignés de l'invention puisqu'ils ne traitent en aucun cas un dispositif de récupération musculaire.

### Exposé de l'invention

La revendication 1 définit l'invention et revendications divulguent des modes de réalisation. La présente invention vise à remédier à tout ou partie de ces inconvénients.

À cet effet, selon un premier aspect, la présente invention vise un dispositif nomade et autonome de récupération musculaire par cryothérapie, qui comporte :
- un récipient de fluide sous pression,
- un détendeur du fluide sous pression, en connexion fluidique avec le récipient et muni d'une sortie de gaz détendu,
- une plaque de matériau thermiquement conducteur configurée pour s'appuyer sur la peau de l'utilisateur et munie d'un radiateur échangeur de chaleur positionné sur le chemin du gaz détendu sortant du détendeur de fluide.

La détente du fluide provoque un refroidissement important du gaz détendu s'échappant du détendeur, ce gaz se réchauffant au contact du radiateur et refroidissant le radiateur et la plaque en appuie sur la peau de l'utilisateur. Le dispositif objet de l'invention ne nécessite aucun moyen externe pour fonctionner et peut avoir des dimensions et un poids similaires à ceux d'une bouteille d'eau. Il est donc nomade et aisément transportable.

Dans des modes de réalisation, le dispositif comporte, de plus, un moyen de maintien amovible de la plaque en position contre un membre d'un utilisateur.

Grâce à ces dispositions, le dispositif peut être maintenu en place en regard d'un muscle d'un utilisateur sans effort de la part de l'utilisateur.

Dans des modes de réalisation, le moyen de maintien amovible comporte au moins une bande de tissu autoagrippant.

Grâce à ces dispositions, aucune manipulation compliquée de l'utilisateur n'est nécessaire.

Dans des modes de réalisation, le dispositif comporte, de plus, un moyen de commande du passage de fluide entre le récipient et le détendeur, le moyen de commande étant configuré pour limiter le débit moyen, pendant la durée d'utilisation, de fluide sortant du récipient à une valeur inférieure au débit maximal possible.

Grâce à ces dispositions, on évite les risques d'un refroidissement trop intense qui pourrait provoquer une « brûlure » de la peau de l'utilisateur.

Dans des modes de réalisation, le dispositif comporte au moins un capteur d'une température représentative de la température de la plaque au contact avec la peau, le moyen de commande étant configuré pour asservir le débit de fluide sortant du récipient à la température captée.

On pilote ainsi la température appliquée à la peau de l'utilisateur, quelle que soit la température extérieure et, éventuellement, pour faire suivre à cette température appliquée à la peau une courbe de refroidissement progressive et prédéterminée.

Dans des modes de réalisation, le récipient comporte une valve qui s'ouvre par décalage angulaire, le moyen de commande comportant un servomoteur en appui sur le détendeur et le moyen de commande étant configuré pour commander le mouvement du servomoteur.

Dans des modes de réalisation, le récipient comporte une valve qui s'ouvre par translation vers le récipient, le moyen de commande comportant un servomoteur en appui sur le détendeur et le moyen de commande étant configuré pour commander le mouvement du servomoteur.

Dans des modes de réalisation, la plaque de matériau thermiquement conducteur comporte de l'aluminium.

Dans des modes de réalisation, la plaque de matériau thermiquement conducteur présente une forme concave.

Dans des modes de réalisation, le fluide sous pression dans le récipient est du hydrofluoro-oléfines sous forme de gaz comprimé.

Les HFO (HydroFluoro-Oléfines) sont des fluides frigorigènes de quatrième génération. Ils présentent des alternatives à faible GWP qui réduisent l'impact environnemental tout en proposant une efficacité énergétique.

Selon un deuxième aspect, l'invention concerne un connecteur mécanique pour un dispositif objet de l'invention, configuré pour maintenir en position un récipient de fluide sous pression dans un compartiment du dispositif de telle manière que le détendeur soit en connexion fluidique avec le récipient.

Dans des modes de réalisation, le connecteur mécanique présente une forme générale cylindrique à directrice circulaire, une ouverture centrale et des ergots radiaux s'étendant au-delà de la génératrice de cette forme cylindrique.

Dans des modes de réalisation, le connecteur mécanique présente, sur la surface intérieure de l'ouverture des portions de sphères.

Dans des modes de réalisation, le connecteur mécanique comporte des entailles radiales dans sa paroi cylindrique, entailles qui définissent des clips présentant des languettes rentrantes orientées vers l'axe central du connecteur, entailles dont l'extrémité libre présente une forme chanfreinée.

Les avantages, buts et caractéristiques particulières de ce connecteur mécanique étant similaires à ceux du dispositif objet de l'invention, ils ne sont pas rappelés ici.

Selon un troisième aspect, qui ne forme pas parti de l' invention, un procédé exemplaire de récupération musculaire par cryothérapie, comporte :
- une étape de positionnement d'une plaque de matériau thermiquement conducteur configurée pour s'appuyer sur la peau de l'utilisateur et munie d'un radiateur échangeur de chaleur,
- une étape de détente d'un fluide sous pression contre la plaque.

Dans des modes de réalisation, le procédé objet de la présente invention comporte, de plus :
- une étape de mesure de la température de la plaque,
- une étape de comparaison de la température mesurée à une température limite prédéterminée,
procédé dans lequel l'étape de détente est mise en œuvre en fonction du résultat de l'étape de comparaison.

Les avantages, buts et caractéristiques particulières du procédé étant similaires à ceux du dispositif objet de l'invention, ils ne sont pas rappelés ici.

### Brève description des figures

D'autres avantages, buts et caractéristiques particulières de l'invention ressortiront de la description non limitative qui suit d'au moins un mode de réalisation particulier du dispositif, du procédé et du kit objets de la présente invention, en regard des dessins annexés, dans lesquels :
- La figure 1 représente, schématiquement, en perspective et en coupe, un premier mode de réalisation particulier du dispositif,
- La figure 2 représente, schématiquement, en vue de côté, le dispositif illustré en figure 1,
- La figure 3 représente, schématiquement, en vue de dessus, le dispositif illustré en figure 1,
- La figure 4 représente, schématiquement, une courbe de température observée avec un dispositif objet de l'invention,
- La figure 5 représente, sous forme d'un logigramme, des étapes d'un mode de réalisation particulier du procédé exemplaire, utilisant l'invention,
- La figure 6 représente, en vue de dessous, un adaptateur de récipient sur le dispositif objet de l'invention,
- La figure 7 représente, en vue de dessus, l'adaptateur illustré en figure 6,
- La figure 8 représente, en vue de côté, l'adaptateur illustré en figures 6 et 7,
- La figure 9 représente, une vue selon une coupe A-A repérée en figures 6 et 8, de dessus, de l'adaptateur illustré en figures 6 à 8,
- La figure 10 représente une vue de détail repérée C en figure 8,
- La figure 11 représente une vue de détail repérée B en figure 9 et
- La figure 12 représente, schématiquement, un adaptateur objet de l'invention dans une vue partielle du récipient et du dispositif objet de l'invention.

### Description des modes de réalisation

La présente description est donnée à titre non limitatif, chaque caractéristique d'un mode de réalisation pouvant être combinée à toute autre caractéristique de tout autre mode de réalisation de manière avantageuse.

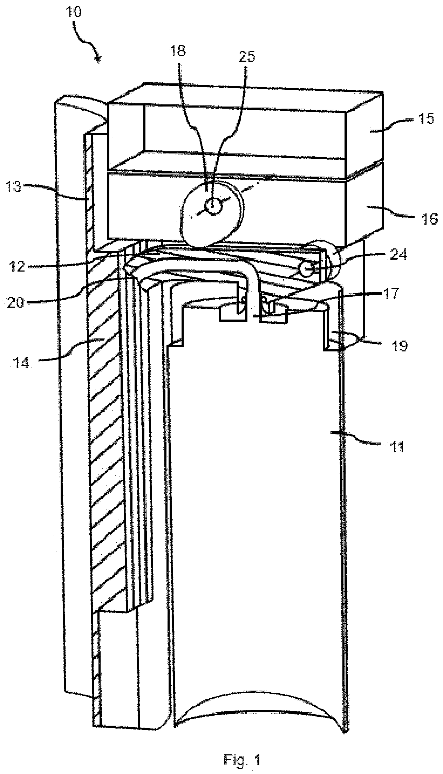

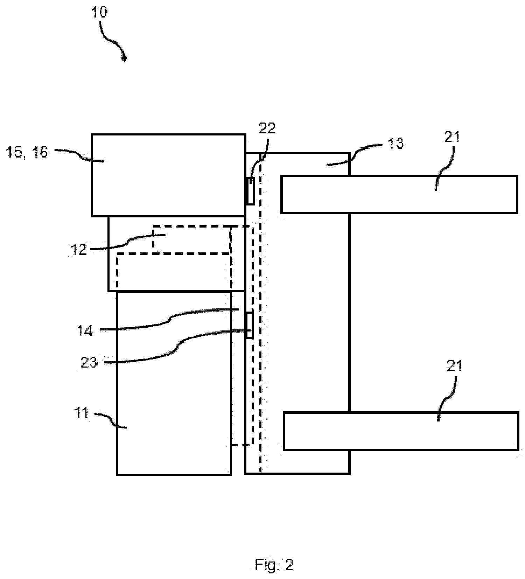

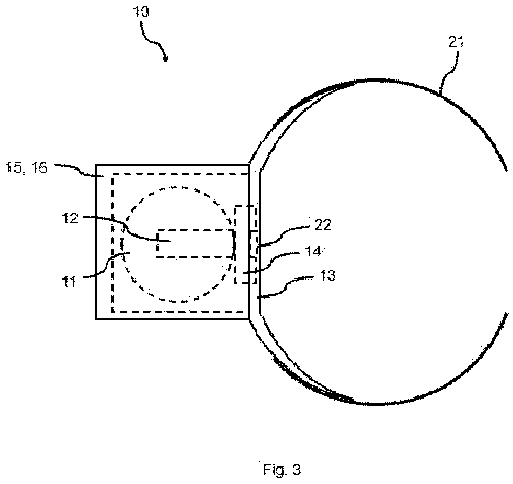

Dans toute la description, on appelle « supérieur » ou « haut » ce qui est en haut dans les figures 1 et 2 et « inférieur » ou « bas » ce qui est en bas dans ces figures. Les figures 1 et 2 représentent l'orientation du dispositif en cours d'utilisation. Les « hauteurs » découlent de ces définitions. Les « largeurs » sont définies de haut en bas dans la figure 3, qui représente une vue de dessus du dispositif. On appelle « interne » ou « intérieur » ce qui est proche ou tourné vers le récipient de fluide sous pression et « externe » ou « extérieur », ce qui est éloigné de ce récipient ou tourné vers l'extérieur du dispositif.

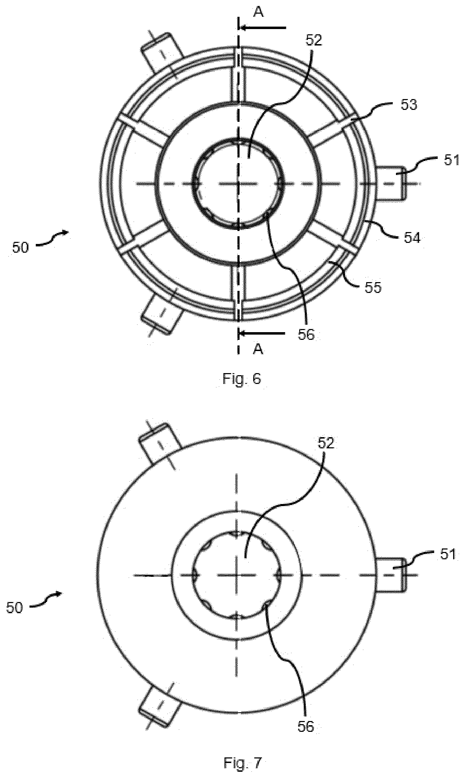

On note dès à présent que les figures 6 à 11 sont à l'échelle, mais que les autres figures ne sont pas à l'échelle.

On observe, sur les figures 1 à 3, un mode de réalisation particulier 10 du dispositif nomade et autonome de récupération musculaire par cryothérapie objet de l'invention. Le dispositif 10 comporte, préférentiellement de manière amovible à la main, sans rupture d'une pièce, un récipient 11 de fluide sous pression. Ce fluide peut être un liquide. Cependant, préférentiellement, ce fluide est un gaz sous pression. Plus préférentiellement, le gaz sous pression dans le récipient 11 est du hydrofluoro-oléfines (« HFO »).

Le dispositif 10 comporte aussi un détendeur 12 du fluide sous pression, en connexion fluidique avec le récipient 11 et muni d'une sortie 20 de gaz détendu. Du fait des lois de la thermodynamique, le gaz détendu est beaucoup plus froid que le fluide contenu dans le récipient 11.

Le dispositif 10 comporte encore une plaque 13 de matériau thermiquement conducteur configurée pour s'appuyer sur la peau de l'utilisateur et muni d'un radiateur échangeur de chaleur 14 positionné sur le chemin du gaz détendu sortant du détendeur de fluide 12. Le radiateur 14 présente, par exemple, des ailettes métalliques parallèles verticales entre lesquelles circule le gaz détendu provenant de la sortie 20 du détendeur 12.

Le gaz très froid s'échappant par la sortie 20 du détendeur 12 se réchauffe au contact du radiateur 14 et refroidit le radiateur 14 et la plaque 13 en appui sur la peau de l'utilisateur. La plaque 13 peut ainsi avoir une température inférieure ou égale à 0°C. Le dispositif 10 ne nécessite aucun moyen externe pour fonctionner et peut avoir des dimensions et un poids similaire à ceux d'une bouteille d'eau. Il est donc nomade et aisément transportable.

Dans des modes de réalisation (non représentés), l'utilisateur peut actionner luimême le détendeur 12 pour provoquer le refroidissement de la plaque 13. Cependant, un tel fonctionnement pourrait s'avérer dangereux, car de trop basses températures peuvent provoquer des « brûlures » de la peau.

Dans le mode de réalisation représenté dans les figures 1 à 3, le dispositif 10 comporte un moyen de commande 16 du passage de fluide entre le récipient 11 et le détendeur 12. Le moyen de commande 16, préférentiellement à contrôleur et mémoire de programme, est configuré pour limiter le débit moyen, pendant la durée d'utilisation, de fluide sortant du récipient 11, à une valeur inférieure au débit maximal possible. Une batterie 15 alimente électriquement le moyen de commande 16.

Pour commander le débit de fluide détendu en sortie 20 du détendeur 12, le moyen de commande 16 commande l'ouverture de la valve 17 du récipient 11.

Dans le dispositif 10, le récipient 11 comporte une valve 17 qui s'ouvre par décalage angulaire et le moyen de commande 16 comporte un servomoteur 18 en appui sur le détendeur 12. Le détendeur 12 est mobile en rotation autour d'un axe 24 et la came du servomoteur 18, de forme oblongue, est mobile en rotation autour d'un axe 25. Le moyen de commande 16 est configuré pour commander le mouvement du servomoteur 18.

Dans des modes de réalisation (non représentés), le récipient comporte une valve qui s'ouvre par translation vers le récipient, le moyen de commande comportant un servomoteur en appui sur le détendeur. Le moyen de commande est alors aussi configuré pour commander le mouvement du servomoteur.

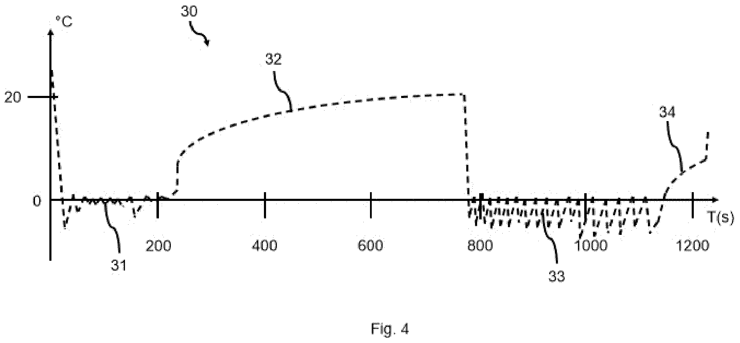

Préférentiellement, le dispositif 10 comporte au moins un capteur 22 ou 23 d'une température représentative de la température de la plaque 13 au contact avec la peau. Dans ce cas, le moyen de commande 16 est configuré pour asservir le débit de fluide en sortie 20 du détendeur 13 à au moins une température captée. On pilote ainsi la température appliquée par la plaque 13 à la peau de l'utilisateur, quelle que soit la température extérieure et, éventuellement, pour faire suivre à cette température appliquée à la peau une courbe de refroidissement progressive et prédéterminée (voir figure 4). Préférentiellement, au moins deux capteurs de température, par exemple des thermistances, sont positionnés en différents endroits de la plaque 13. Dans les figures, le capteur de température 22 est positionné à proximité d'une extrémité de la plaque 13 et le capteur de température 23 est positionné au centre de la plaque 13.

Un connecteur mécanique ou adaptateur 50 (voir figures 6 à 12), dans un logement 19, maintient en place, de manière amovible à la main sans rupture de pièce, la valve 17 du récipient 11 en regard du détendeur 12. Ce connecteur mécanique 50 est préférentiellement constitué d'un matériau thermiquement isolant.

Pour maintenir le dispositif 10 sur un membre de l'utilisateur, et plus particulièrement contre les muscles de son corps, le dispositif 10 comporte au moins un moyen de maintien de la plaque en position contre un membre d'un utilisateur. Préférentiellement, le moyen de maintien comporte au moins une ceinture 21 (non représenté en figure 1), préférentiellement une bande de tissu autoagrippant, communément appelé « scratch ». Dans des modes de réalisation, le moyen de maintien comporte une bande de ruban adhésif. Dans des modes de réalisation, le moyen de maintien comporte une lanière munie d'une boucle de serrage, d'un loquet rétractable ou d'une butée rétractable.

Le moyen de maintien est configuré pour maintenir la plaque en contact avec la peau d'un utilisateur, au niveau d'au moins un muscle de l'utilisateur. Préférentiellement, le moyen de maintien est configuré pour enserrer un membre de l'utilisateur, c'est-à-dire que le moyen de maintien et au moins une partie de la plaque entourent le membre de l'utilisateur.

Dans des modes de réalisation, la plaque de matériau thermiquement conducteur 13 comporte de l'aluminium. Dans des modes de réalisation, la plaque de matériau thermiquement conducteur 13 présente une forme concave, visible en figures 1 et 3. Cette forme concave, ici cylindrique d'axe vertical, épouse la forme d'un membre de l'utilisateur et augmente la transmission de frigories depuis la plaque 13 au membre considéré.

On note que le dispositif objet de l'invention peut être utilisé en mode cryothérapie et/ou en mode cryothérapie avec compression grâce à au moins un textile de compression, par exemple le textile de compression du sportif.

Selon un aspect, l'invention concerne un élément de kit de récupération musculaire par cryothérapie, qui comporte un détendeur du fluide sous pression muni d'une sortie de gaz détendu mécaniquement lié à une plaque de matériau thermiquement conducteur configurée pour s'appuyer sur la peau de l'utilisateur et munie d'un radiateur échangeur de chaleur positionné sur le chemin du gaz détendu sortant du détendeur de fluide, et un connecteur mécanique configuré pour maintenir en position un récipient de fluide sous pression dans un compartiment de telle manière que le détendeur soit en connexion fluidique avec le récipient.

La figure 4 illustre le déroulement d'une séance de 21 minutes de récupération musculaire par cryothérapie. En abscisse est représentée la température mesurée par un capteur de température, par exemple le capteur 23, en degrés Celsius. En ordonnée est représenté le temps, en secondes. La courbe 30 de température en fonction du temps comporte une première séquence 31, s'étendant de l'instant 0 à environ 230 secondes dont environ 180 secondes avec une température oscillant autour de 0°C, après une baisse de température initiale brutale commandée par le moyen de commande 16 jusqu'à ce que la température mesurée soit inférieure ou égale à 0°C. Le moyen de commande 16 déclenche ensuite une impulsion de détente de gaz à chaque fois que la température mesurée dépasse 0°C. L'inventeur a observé que ces impulsions surveillent sensiblement toutes les 20 secondes. Pendant une deuxième séquence 32 s'étendant sensiblement de la seconde 230 à la seconde 780, aucune détente de gaz n'est commandée par le moyen de commande 16. La troisième séquence 33 est similaire à la première séquence 31, si ce n'est qu'elle s'étend sensiblement de la seconde 780 à la seconde 1140. Au cours de la dernière séquence 34, aucune détente de gaz n'est commandée par le moyen de commande 16.

Pour une température du récipient 11 de 15°C et une température ambiante de 19 °C, il a été observé une température du mollet de 31 °C en début de séance et une température de 15 °C en fin de séance, l'utilisateur ressentant une sensation d'anesthésie locale. Cette séance de récupération consomme environ 90 grammes de gaz comprimé.

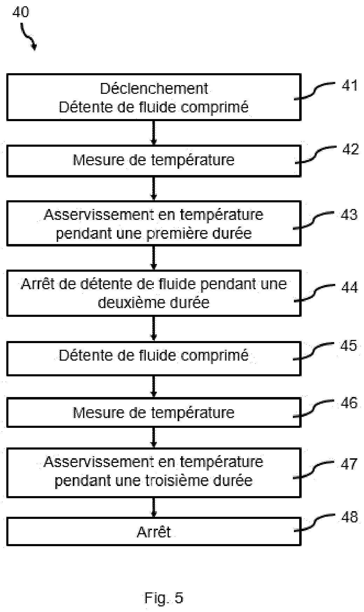

La figure 5 illustre les étapes exemplaires d'un fonctionnement 40 du dispositif objet de l'invention. Au cours d'une étape 41, l'utilisateur, après avoir positionné la plaque 13 contre son muscle à traiter et éventuellement serré le moyen de maintien, déclenche le fonctionnement du dispositif et, notamment l'alimentation électrique du moyen de commande 16 par la batterie 15. Ce moyen de commande 16 provoque la détente de gaz à travers le détendeur 12.

Au cours des étapes 42 et 43, la température de la plaque 13 est mesurée de manière continue. Au cours de l'étape 43 et pendant une première durée (environ 230 secondes en figure 4), le moyen de commande 16 asservit l'ouverture de la valve 17, par l'intermédiaire du servomoteur 18, à la température mesurée. Dès que la température devient inférieure ou égale à 0°C, le moyen de commande 16 arrête la détente de gaz et, dès que la température devient supérieure ou égale à 0°C, le moyen de commande 16 provoque une impulsion de détente de gaz.

Au cours d'une étape 44, à la fin de la première durée, le moyen de commande arrête la détente du gaz pendant une deuxième durée (environ 550 secondes en figure 4).

Au cours d'une étape 45 suivant la fin de l'étape 44, le moyen de commande 16 provoque la détente de gaz. La mesure de température est effectuée de manière continue pendant des étapes 46 et 47. Au cours de l'étape 47 et pendant une troisième durée (environ 360 secondes en figure 4), le moyen de commande 16 asservit l'ouverture de la valve 17, par l'intermédiaire du servomoteur 18, à la température mesurée. Dès que la température devient inférieure à 0°C, le moyen de commande 16 arrête la détente de gaz et, dès que la température devient supérieure à 0°C, le moyen de commande 16 provoque une impulsion de détente de gaz.

Enfin, le dispositif 10 se remet à l'arrêt au cours d'une étape 48.

La présente invention présente les avantages suivants :
- un fonctionnement en continu d'au moins 10 minutes avec un récipient de 100mL plein,
- un fonctionnement possible avec différents formats de récipient pouvant aller de 100 mL à 600 mL,
- un effet anesthésiant,

On observe, en figures 6 à 12, un adaptateur 50 se positionnant en interface mécanique entre le récipient 11 et le dispositif 10. Le connecteur mécanique 50 pour un dispositif 10 objet de l'invention, est configuré pour maintenir en position un récipient 11 de fluide sous pression dans le compartiment 19 du dispositif 10 de telle manière que le détendeur soit en connexion fluidique avec le récipient. L'adaptateur 50 est en matière plastique, par exemple en ABS (acrylonitrile butadiène styrène) ou en PA (polyamide).

L'adaptateur 50 présente une forme générale cylindrique à base circulaire, entre deux plans perpendiculaires aux génératrices, dont la distance est de l'ordre de la moitié du rayon de la forme cylindrique de l'adaptateur 50. Cette forme comporte des excroissances 51 externes et des évidements.

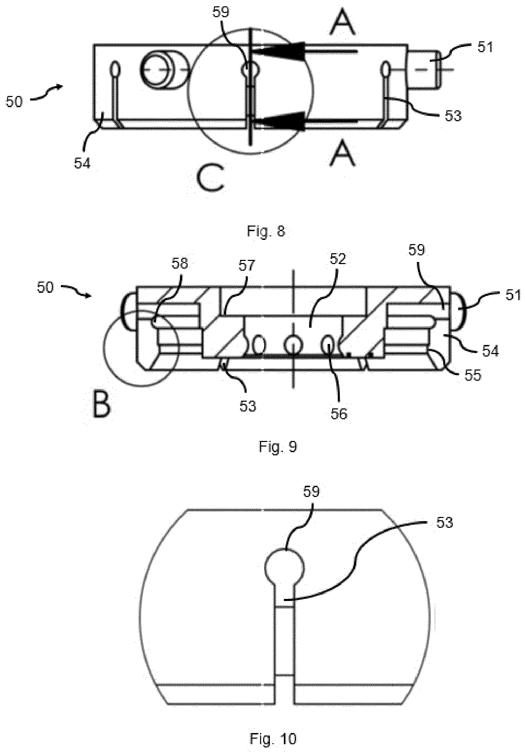

Les excroissances radiales 51 s'étendent perpendiculairement à la génératrice (verticale en figures 8 et 9) s'étendent au-delà de la génératrice de cette forme cylindrique. Ces excroissances ou ergots 51, préférentiellement cylindriques et au nombre de trois, participent à un mouvement de baïonnette : guidage en translation puis en rotation de l'adaptateur 50 dans des rainures ou gorges (non représentées) du corps du dispositif 10, vissage de la tête du récipient 11 dans le corps du dispositif 10 pour mettre la buse 17 du récipient 11 à la bonne hauteur, par rapport à la buse de dégazage ou détendeur 12, immobilisation de la tête du récipient 11 dans le corps du dispositif 10.

Une ouverture centrale traversante 52 permet le passage de la buse 17 du récipient 11 du corps du récipient 11 jusqu'à la buse de dégazage du détendeur 12. L'ouverture centrale 52 présente un épaulement 57. Des portions de sphères 56, présentes sur la face cylindrique intérieure de l'ouverture 52 servent à faire obstacle dans les déformations du sertissage de la valve du récipient 11 et/ou pour empêcher la rotation de l'adaptateur 50 sur le récipient 11.

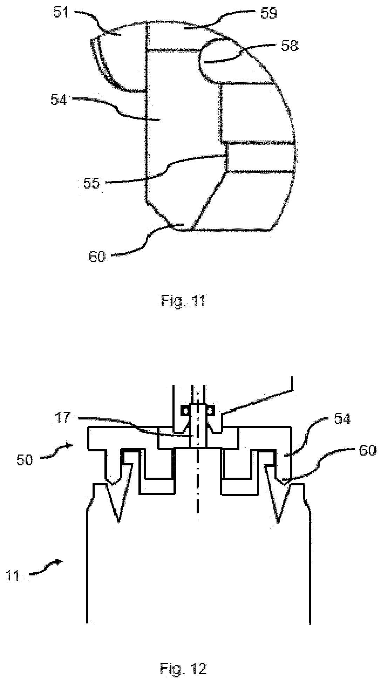

Des entailles radiales 53 (ici au nombre de six) permettent la déformation latérale des clips 54 de l'adaptateur 50 formés par la périphérie de l'adaptateur 50 entre les entailles 53. Ces clips 54 dont la section est représentée en figure 11, présentent :
- des gorges 58 en correspondance avec le sertissage supérieur du corps du récipient 11 et
- des languettes rentrantes 55 qui enserrent la tête du récipient 11 et retiennent mécaniquement le récipient 11 dans le dispositif 10. Ces languettes rentrantes 55 sont orientées vers l'axe central du connecteur 50.

Les entailles 53 présentent préférentiellement une extrémité arrondie 59 pour éviter les concentrations de contraintes, comme illustrées en figure 10. Les clips 54 ont une extrémité libre 60 présentant une forme chanfreinée leur conférant une section triangulaire, pour faciliter la pénétration des clips 54 dans le relief supérieur du récipient 11.

La portée de l'invention est définie par les revendications.

## Revendications

1. Dispositif (10) nomade et autonome de récupération musculaire par cryothérapie, comportant:
- un récipient (11) de fluide sous pression,
- un détendeur (12) du fluide sous pression, en connexion fluidique avec le récipient et muni d'une sortie (20) de gaz détendu,
- une plaque (13) de matériau thermiquement conducteur configurée pour s'appuyer sur la peau de l'utilisateur et munie d'un radiateur (14) échangeur de chaleur positionné sur le chemin du gaz détendu sortant du détendeur de fluide et
- **caractérisé par** un moyen de maintien amovible de la plaque en position contre un membre d'un utilisateur.

2. Dispositif (10) selon la revendication 1, dans lequel le moyen de maintien amovible comporte au moins une bande de tissu autoagrippant (21).

3. Dispositif (10) selon l'une des revendications 1 ou 2 qui comporte, de plus, un moyen de commande (16) du passage de fluide entre le récipient (11) et le détendeur (12), le moyen de commande étant configuré pour limiter le débit moyen, pendant la durée d'utilisation, de fluide sortant du récipient à une valeur inférieure au débit maximal possible.

4. Dispositif (10) selon la revendication 3, qui comporte, de plus, au moins un capteur (22, 23) d'une température représentative de la température de la plaque (13) au contact avec la peau, le moyen de commande (16) étant configuré pour asservir le débit de fluide sortant du récipient (11) à la température captée.

5. Dispositif (10) selon l'une des revendications 3 ou 4, dans lequel le récipient (11) comporte une valve (17) qui s'ouvre par décalage angulaire, le moyen de commande (16) comportant un servomoteur (18) en appui sur le détendeur (12) et le moyen de commande étant configuré pour commander le mouvement du servomoteur.

6. Dispositif selon l'une des revendications 3 ou 4, dans lequel le récipient comporte une valve qui s'ouvre par translation vers le récipient, le moyen de commande comportant un servomoteur en appui sur le détendeur et le moyen de commande étant configuré pour commander le mouvement du servomoteur.

7. Dispositif (10) selon l'une des revendications 1 à 6, dans lequel la plaque (13) de matériau thermiquement conducteur comporte de l'aluminium et/ou présente une forme concave.

8. Dispositif (10) selon l'une des revendications 1 à 7, dans lequel le fluide sous pression dans le récipient (11) est du hydrofluoro-oléfines sous forme de gaz comprimé.

9. Dispositif (10) selon l'une des revendications 1 à 8, comprenant un connecteur mécanique (50), configuré pour maintenir en position un récipient (11) de fluide sous pression dans un compartiment (19) du dispositif (10) de telle manière que le détendeur soit en connexion fluidique avec le récipient.

10. Dispositif (10) selon la revendication 9, dans lequel le connecteur mécanique présente une forme générale cylindrique à directrice circulaire, une ouverture centrale (52) et des ergots radiaux (51) s'étendant au-delà de la génératrice de cette forme cylindrique.

11. Dispositif (10) selon la revendication 10, dans lequel le connecteur mécanique présente, sur la surface intérieure de l'ouverture (52), des portions de sphères (56).

12. Dispositif (10) selon l'une des revendications 9 à 11, dans lequel le connecteur mécanique comporte des entailles radiales (53) dans sa paroi cylindrique, entailles qui définissent des clips (54) présentant des languettes rentrantes (55) orientées vers l'axe central du connecteur, entailles dont l'extrémité libre (60) présente une forme chanfreinée.

## Patentansprüche

1. Mobile und autonome Vorrichtung (10) zur Muskelerholung durch Kryotherapie, aufweisend:
- einen Behälter (11) für unter Druck stehendes Fluid,
- einen Druckminderer (12) des unter Druck stehenden Fluids, der mit dem Behälter fluidisch verbunden ist und mit einem Ausgang (20) für entspanntes Gas ausgestattet ist,
- eine Platte (13) aus wärmeleitendem Material, die so eingerichtet ist, dass sie auf der Haut des Benutzers aufliegt und mit einem Wärmetauscher (14) versehen ist, der auf dem Weg des aus dem Fluiddruckminderer austretenden entspannten Gases positioniert ist, und
**gekennzeichnet durch** ein abnehmbares Mittel zum Halten der Platte in Position an einem Körperglied eines Benutzers.

2. Vorrichtung (10) nach Anspruch 1, wobei das abnehmbare Haltemittel mindestens einen Klettverbingungsstreifen (21) aufweist.

3. Vorrichtung (10) nach einem der Ansprüche 1 oder 2, die ferner ein Steuermittel (16) für den Fluiddurchgang zwischen dem Behälter (11) und dem Druckminderer (12) aufweist, wobei das Steuermittel so eingerichtet ist, dass es den durchschnittlichen Durchfluss von Fluid, das während der Nutzungsdauer aus dem Behälter austritt, auf einen Wert begrenzt, der unter dem maximal möglichen Durchfluss liegt.

4. Vorrichtung (10) nach Anspruch 3, die ferner mindestens einen Sensor (22, 23) einer Temperatur aufweist, die repräsentativ für die Temperatur der Platte (13) in Kontakt mit der Haut ist, wobei das Steuermittel (16) so eingerichtet ist, dass es den aus dem Behälter (11) austretenden Fluidfluss auf die erfasste Temperatur regelt.

5. Vorrichtung (10) nach einem der Ansprüche 3 oder 4, wobei der Behälter (11) ein Ventil (17) aufweist, das sich durch Winkelverschiebung öffnet, wobei das Steuermittel (16) einen Servomotor (18) aufweist, der auf dem Druckminderer (12) aufliegt, und das Steuermittel so eingerichtet ist, dass es die Bewegung des Servomotors steuert.

6. Vorrichtung nach einem der Ansprüche 3 oder 4, wobei der Behälter ein Ventil aufweist, das sich durch Translation zum Behälter öffnet, wobei das Steuermittel einen Servomotor aufweist, der auf dem Druckminderer aufliegt, und das Steuermittel so eingerichtet ist, dass es die Bewegung des Servomotors steuert.

7. Vorrichtung (10) nach einem der Ansprüche 1 bis 6, wobei die Platte (13) aus wärmeleitendem Material Aluminium aufweist und/oder eine konkave Form besitzt.

8. Vorrichtung (10) nach einem der Ansprüche 1 bis 7, wobei das unter Druck stehende Fluid in dem Behälter (11) Hydrofluorolefine in Form eines komprimierten Gases ist.

9. Vorrichtung (10) nach einem der Ansprüche 1 bis 8, umfassend einen mechanischen Verbinder (50), der so eingerichtet ist, dass er einen Behälter (11) mit unter Druck stehendem Fluid in einem Fach (19) der Vorrichtung (10) derart in Position hält, dass der Druckminderer in fluidischer Verbindung mit dem Behälter steht.

10. Vorrichtung (10) nach Anspruch 9, wobei der mechanische Verbinder eine zylindrische allgemeine Form mit kreisförmiger Leitlinie, eine zentrale Öffnung (52) und radiale Nasen (51) aufweist, die sich über die Generatrix dieser zylindrischen Form hinaus erstrecken.

11. Vorrichtung (10) nach Anspruch 10, wobei der mechanische Verbinder an der Innenfläche der Öffnung (52) Kugelabschnitte (56) besitzt.

12. Vorrichtung (10) nach einem der Ansprüche 9 bis 11, wobei der mechanische Verbinder in seiner zylindrischen Wand radiale Einkerbungen (53) aufweist, welche Einkerbungen die Clips (54) definieren, die einfahrende Laschen (55) aufweisen, die zur Mittelachse des Verbinders ausgerichtet sind, Einkerbungen, deren freies Ende (60) eine abgeschrägte Form aufweist.

## Claims

1. Portable stand-alone device (10) for muscle recovery by means of cryotherapy, comprising:
- a container (11) of pressurised fluid,
- a pressure reducer (12) for the pressurised fluid, being fluidly connected to the container and fitted with an outlet (20) for expanded gas,
- a plate (13) made of thermally conductive material designed to rest against the user's skin and equipped with a heat exchanger radiator (14) positioned in the path of the expanded gas exiting the fluid pressure reducer and
**characterised by** a removable means for securing the plate in position against a user's limb.

2. Device (10) according to claim 1, wherein the removable securing means comprises at least one strip of hook-and-loop fabric (21).

3. Device (10) according to any one of claims 1 or 2, which additionally comprises a control means (16) for regulating the flow of fluid between the container (11) and the pressure reducer (12), the control means being configured to limit the average flow rate, for the duration of use, of fluid exiting the container to a value lower than the maximum possible flow rate.

4. Device (10) according to claim 3, which additionally comprises at least one sensor (22, 23) for a temperature representative of the temperature of the plate (13) in contact with the skin, the control means (16) being configured to regulate the flow of fluid exiting the container (11) depending on the detected temperature.

5. Device (10) according to any one of claims 3 or 4, wherein the container (11) comprises a valve (17) that opens by angular offset, the control means (16) comprising a servomotor (18) resting on the pressure reducer (12), and the control means being configured to control the movement of the servomotor.

6. Device according to any one of claims 3 or 4, wherein the container comprises a valve that opens by translation towards the container, the control means comprising a servomotor resting on the pressure reducer and the control means being configured to control the movement of the servomotor.

7. Device (10) according to any one of claims 1 to 6, wherein the plate (13) made of thermally conductive material includes aluminium and/or has a concave shape.

8. Device (10) according to any one of claims 1 to 7, wherein the pressurised fluid in the container (11) is a hydrofluoroolefin in the form of a compressed gas.

9. Device (10) according to any one of claims 1 to 8, comprising a mechanical connector (50), configured to hold in position a container (11) of pressurised fluid in a compartment (19) of the device (10) such that the pressure reducer is fluidly connected to the container.

10. Device (10) according to claim 9, wherein the mechanical connector has a generally cylindrical shape with a circular directrix, a central opening (52), and radial lugs (51) extending beyond the generatrix of this cylindrical shape.

11. Device (10) according to claim 10, wherein the mechanical connector has portions of spheres (56) on the inner surface of the opening (52).

12. Device (10) according to one of claims 9 to 11, wherein the mechanical connector comprises radial notches (53) in its cylindrical wall, which notches define clips (54) having retracting tabs (55) oriented towards the central axis of the connector, notches whose free end (60) has a chamfered shape.
